# EUROPEAN PATENT APPLICATION

(11) **EP 4 803 083 A1**
(43) Date of publication of application: **09.09.2026**
(21) Application number: 24884345.0
(22) Date of filing: 29.09.2024
(51) Int. Cl.: A61K 31/53, A61K 31/513, A61K 31/706, A61P 1/16, A61P 11/00

(54) **USE OF PYRROLOTRIAZINE COMPOUND IN PREPARATION OF DRUG FOR PREVENTING AND/OR TREATING FIBROSIS OR RELATED DISEASE THEREOF**

(30) Priority: 03.11.2023 CN 202311469656
(71) Applicant: Jumbo Drug Bank Co., Ltd., High-tech Zone Chengdu Sichuan 610000 (CN)
(72) Inventor: WEI, Xiawei, Chengdu, Sichuan 610000 (CN); WEI, Yuquan, Chengdu, Sichuan 610000 (CN); JIANG, Ning, Chengdu, Sichuan 610000 (CN)
(74) Representative: Gille Hrabal Partnerschaftsgesellschaft mbB Patentanwälte
(86) International application number: PCT/CN2024/122312
(87) International publication number: WO 2025/092340

(57) **Abstract**

The present disclosure relates to use of a compound of formula (I) or a pharmaceutically acceptable salt thereof in the preparation of a medicament for preventing and/or treating fibrosis or a related disease thereof.

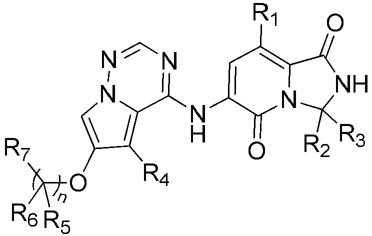

## Description

The present disclosure claims priority to the prior application with the patent application No. 202311469656.3 and entitled "USE OF PYRROLOTRIAZINE COMPOUND IN PREPARATION OF DRUG FOR PREVENTING AND/OR TREATING FIBROSIS OR RELATED DISEASE THEREOF" filed to China National Intellectual Property Administration on November 3, 2023, which is incorporated herein by reference in its entirety.

### TECHNICAL FIELD

The present disclosure belongs to the pharmaceutical field, and specifically relates to the application of pyrrolotriazine compounds in the preparation of a medicament for preventing and/or treating fibrosis or related diseases.

### BACKGROUND

Fibrosis is the formation of scar tissue and tissue stiffening caused by excessive deposition of extracellular matrix (ECM) proteins by myofibroblasts during chronic inflammatory responses. Various harmful stimuli (including toxins, infectious pathogens, autoimmune reactions, and mechanical stress) can induce fibrotic cellular responses.

In response to tissue injury, myofibroblasts derived from multiple sources (including resident fibroblasts, mesenchymal cells, circulating fibrocytes, and transdifferentiation of other cell types) can initiate a wound-healing response by remodeling the extracellular environment to restore tissue integrity and promote replacement of parenchymal cells. Typically, when tissue healing occurs, this pro-fibrotic program is shut down. However, persistent injury and damage lead to dysregulation of this process, resulting in pathological excessive deposition of ECM proteins, accompanied by upregulated myofibroblast activity, creating a chronic inflammatory environment with infiltration of macrophages and immune cells. In this cellular environment, cytokines and growth factors are released in large quantities, ultimately leading to upregulated expression of target genes whose functions further enhance myofibroblast differentiation and the production and secretion of ECM proteins (including collagen, laminin, and fibronectin). As excessive ECM deposition progresses, the structure of the matrix changes and becomes stiff.

Fibrosis is a quasi-tumorous lesion between benign and malignant; it often occurs in organs and tissues such as the liver, kidneys, heart, lungs, and bone marrow, can affect nearly every organ, and ultimately induces multi-organ failure and carcinogenesis, seriously endangering life and health.

Pulmonary fibrosis can generally be classified by cause into idiopathic, primary, immune-related, drug-induced, and physicochemical factor-related types. Idiopathic pulmonary fibrosis accounts for the highest proportion among them.

Idiopathic pulmonary fibrosis (IPF) is a chronic fibrosing interstitial pneumonia of unknown cause that mainly occurs in the elderly and is characterized by dyspnea and progressive deterioration of lung function. The abnormal accumulation of fibrotic tissue in the lung parenchyma severely affects respiratory function, presenting as dry cough and progressive dyspnea (a subjective feeling of insufficient air), and as the disease and lung injury worsen, the patient's respiratory function continues to deteriorate.

The cause and mechanism of IPF remain unclear to date, and treatment goals are to relieve symptoms, improve quality of life, slow or stop disease progression, and increase survival rate. Prednisone, azathioprine, and N-acetylcysteine (NAC) have been used to treat symptoms associated with IPF, but they usually do not significantly increase life expectancy.

Nonalcoholic steatohepatitis (NASH) is a disease mainly characterized by inflammation and fibrosis and can progress to cirrhosis, liver failure, and other conditions. Nowadays, NASH is rapidly becoming the leading cause of liver transplantation.

WO2021098691A1 discloses a class of pyrrolotriazine compounds as MNK inhibitors. Further studies of the present disclosure have found that this class of compounds is expected to be used in the treatment of fibrotic diseases.

### SUMMARY

The present disclosure provides the use of a compound of formula (I) or a pharmaceutically acceptable salt thereof in the preparation of a medicament for the prevention and/or treatment of fibrosis or related diseases or conditions, 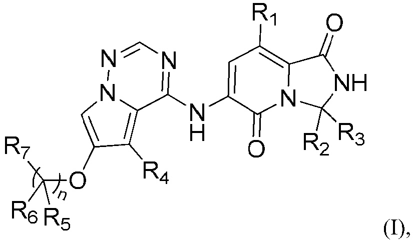
wherein R₁ is selected from H, F, Cl, Br, or C₁₋₃ alkyl;
R₂ and R₃ are each independently selected from H or C₁₋₃ alkyl, wherein the C₁₋₃ alkyl is optionally substituted with 1, 2, or 3 substituents independently selected from F, Cl, Br, or I;
or R₂ and R₃ together with the carbon atom to which they are attached form cyclopentyl, cyclohexyl, or piperidinyl, wherein the cyclopentyl, cyclohexyl, and piperidinyl are optionally substituted with 1, 2, or 3 Rₐ;
each Rₐ is independently selected from H, F, Cl, Br, or C₁₋₃ alkyl;
R₄ is selected from H, F, Cl, Br, or C₁₋₃ alkyl;
R₅ and R₆ are each independently selected from H, F, Cl, Br, I, or C₁₋₃ alkyl;
R₇ is pyrrolidinyl, wherein the pyrrolidinyl is optionally substituted with 1, 2, or 3 R_{b};
each R_{b} is independently selected from H, F, Cl, Br, I, or C₁₋₃ alkyl, wherein the C₁₋₃ alkyl is optionally substituted with 1, 2, or 3 substituents independently selected from F, Cl, Br, or I;
n is 1 or 2.

In some embodiments of the present disclosure, each Rₐ above is independently selected from H, F, Cl, Br, - CH₃, or -CH₂CH₃, and other variables are as defined in the present disclosure.

In some embodiments of the present disclosure, R₂ and R₃ above are each independently selected from H, -CH₃, or -CH₂CH₃, and other variables are as defined in the present disclosure.

In some embodiments of the present disclosure, R₂ and R₃ above together with the carbon atom to which they are attached form 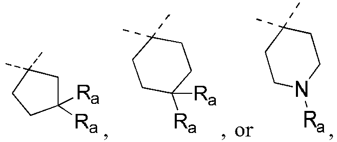 and Rₐ and other variables are as defined in the present disclosure.

In some embodiments of the present disclosure, R₂ and R₃ above together with the carbon atom to which they are attached form 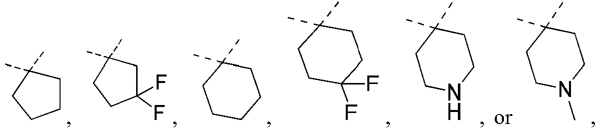 and other variables are as defined in the present disclosure.

In some embodiments of the present disclosure, the above structural unit 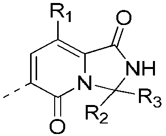 is selected from 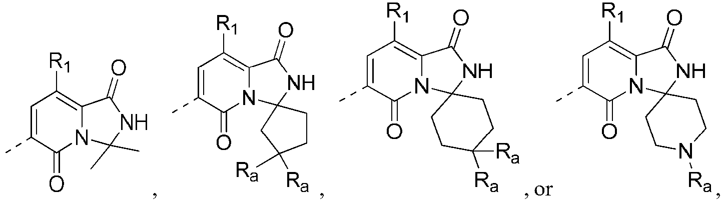 and R₁, Rₐ, and other variables are as defined in the present disclosure.

In some embodiments of the present disclosure, the above structural unit 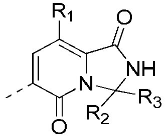 is selected from 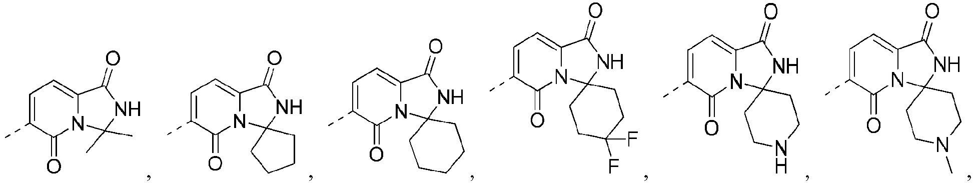 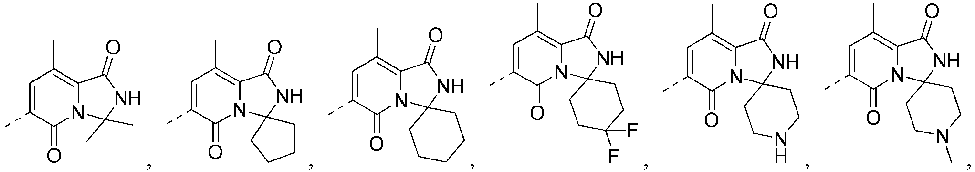 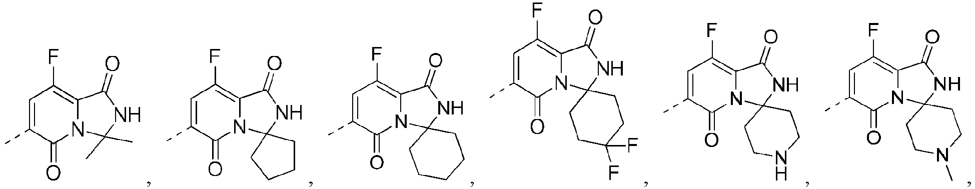 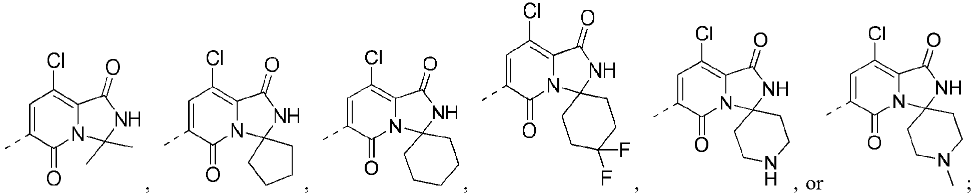 and other variables are as defined in the present disclosure.

In some embodiments of the present disclosure, R₁ is C₁₋₃ alkyl, such as methyl.

In some embodiments of the present disclosure, R₂ and R₃ together with the carbon atom to which they are attached form

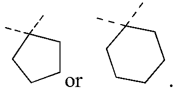

In some embodiments of the present disclosure, R₄ is C₁₋₃ alkyl, such as methyl.

In some embodiments of the present disclosure, R₅ and R₆ are each independently selected from H or methyl; n is 2;
In some embodiments of the present disclosure,

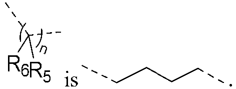

In some embodiments of the present disclosure, R₇ is 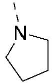 substituted with 1, 2, or 3 H, F, Cl, or methyl, for example

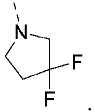

In some embodiments of the present disclosure, the above compound has a structure as shown in any of structural formulas (I-1) ~ (I-4): 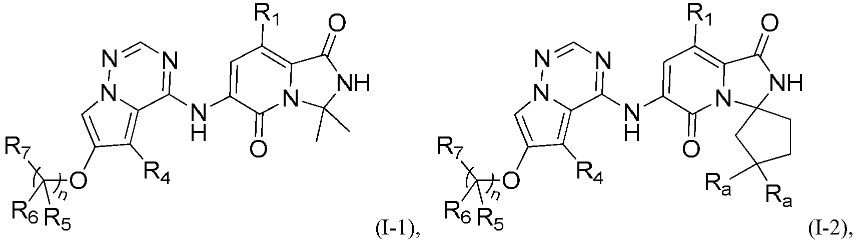 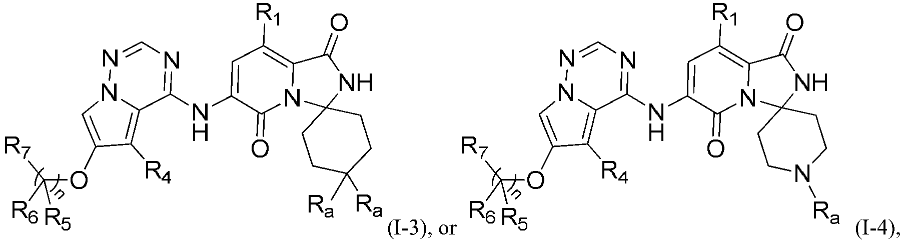 wherein R₁, R₄, R₅, R₆, R₇, Rₐ, and n are as defined in the present disclosure.

In some embodiments of the present disclosure, each of the above R_{b} is independently selected from H, F, Cl, Br, I, 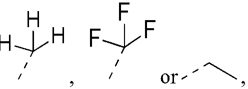 wherein other variables are as defined in the present disclosure.

In some embodiments of the present disclosure, the above R₇ is 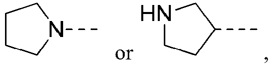 wherein 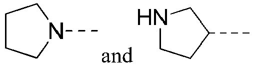 are optionally substituted by 1 or 2 R_{b}, wherein R_{b} and other variables are as defined in the present disclosure.

In some embodiments of the present disclosure, the above R₇ is 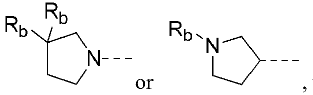 wherein R_{b} and other variables are as defined in the present disclosure.

In some embodiments of the present disclosure, the above R₇ is 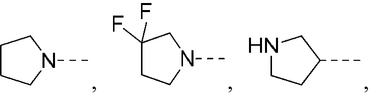 or 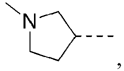 and other variables are as defined in the present disclosure.

In some embodiments of the present disclosure, the above R₄ is selected from H or -CH₃, and other variables are as defined in the present disclosure.

In some embodiments of the present disclosure, the above compound has a structure as shown in any of structural formulas (I-5) ~ (I-9): 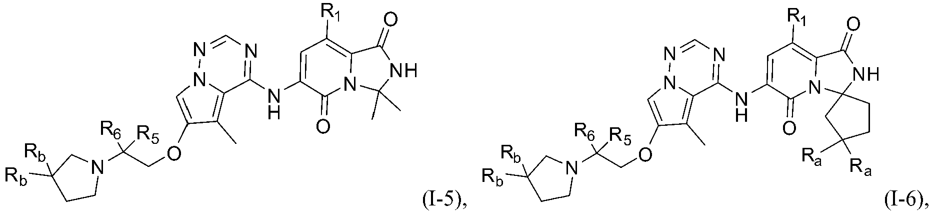 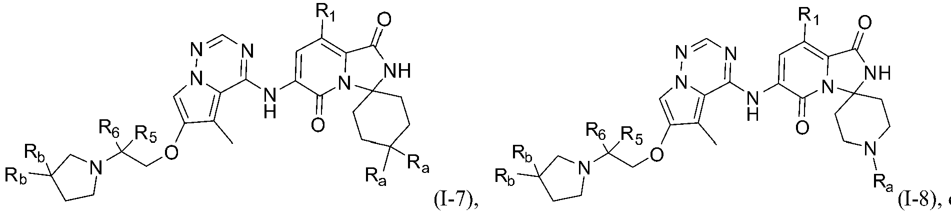 or 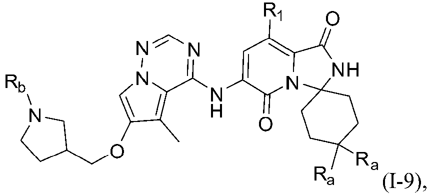 wherein R₁, R₅, R₆, Rₐ, and R_{b} are as defined in the present disclosure.

In some embodiments of the present disclosure, the above R₁ is selected from H, F, Cl, or 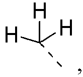 and other variables are as defined in the present disclosure.

In some embodiments of the present disclosure, each of the above R₅ and R₆ is independently selected from H or 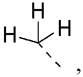 and other variables are as defined in the present disclosure.

Some embodiments of the present disclosure are also derived from any combination of the above variables.

According to embodiments of the present disclosure, the compound represented by formula (I) is selected from the following structures:

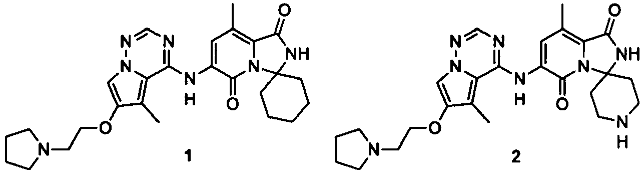

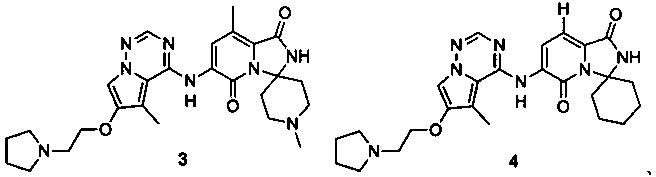

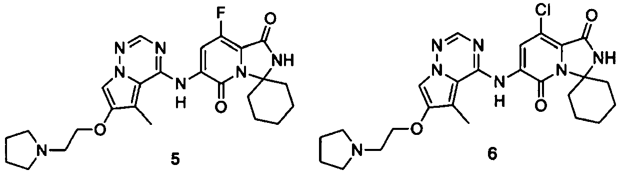

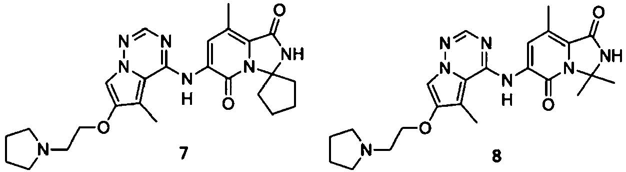

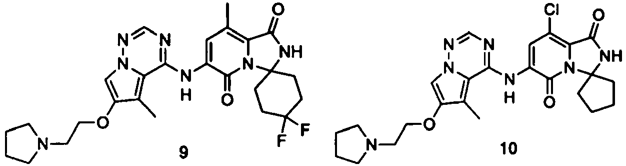

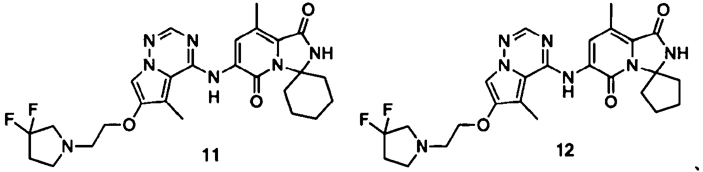

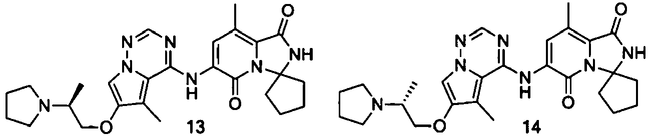

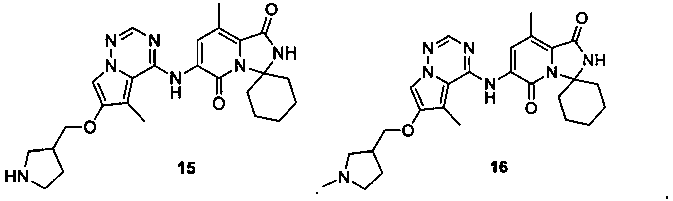

According to embodiments of the present disclosure, the pharmaceutically acceptable salt is a salt formed by the compound represented by formula (I) with an inorganic acid, wherein the inorganic acid includes, for example, hydrochloric acid, hydrobromic acid, nitric acid, carbonic acid, bicarbonate, phosphoric acid, hydrogen phosphate, dihydrogen phosphate, sulfuric acid, hydrogen sulfate, hydroiodic acid, phosphorous acid, and the like; as well as organic acid salts, wherein the organic acids include acids such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; also included are salts of amino acids (such as arginine, etc.), as well as salts of organic acids such as glucuronic acid; preferably the hydrochloride salt or p-toluenesulfonate salt of the compound represented by formula (I).

According to embodiments of the present disclosure, the fibrosis or related disease or condition is selected from pulmonary fibrosis, renal fibrosis, myelofibrosis, cystic fibrosis, oral mucosal fibrosis, hepatic fibrosis, biliary fibrosis, myocardial fibrosis, skin fibrosis, ocular fibrosis, and pancreatic fibrosis.

In some embodiments, the fibrosis or related disease or condition is selected from inflammatory diseases, for example selected from pneumonia, hepatitis, nephritis, myocarditis, and pancreatitis.

In some embodiments, the fibrosis or related disease or condition is selected from liver-related diseases, for example hepatitis, liver cirrhosis, liver injury, or liver failure.

In some embodiments, the fibrosis or related disease or condition is selected from nonalcoholic fatty liver disease or nonalcoholic steatohepatitis (NASH).

In some embodiments, the fibrosis or related disease or condition is selected from progressive fibrosing interstitial lung disease (PF-ILD), particularly diseases presenting pulmonary fibrosis manifestations, such as idiopathic pulmonary fibrosis (IPF), systemic sclerosis-associated ILD (SSc-ILD), connective tissue disease-associated ILD (CTD-ILD), rheumatoid arthritis-associated ILD (RA-ILD), chronic fibrosing hypersensitivity pneumonitis (HP), idiopathic nonspecific interstitial pneumonia (iNSIP), unclassifiable idiopathic interstitial pneumonia (IIP), environmental/occupational fibrotic lung disease, interstitial pneumonia with autoimmune features (IPAF), and sarcoidosis.

In some embodiments, the fibrosis or related disease or condition is selected from muscular dystrophy, fibromatosis, and myelofibrosis, preferably selected from Duchenne muscular dystrophy, Dupuytren's contracture, and primary myelofibrosis (PMF).

The present disclosure also provides a method for preventing and/or treating fibrosis or related diseases or conditions thereof, the method comprising administering to a patient a therapeutically effective amount of the compound represented by formula (I) or a pharmaceutically acceptable salt thereof.

When preparing the pharmaceutical of the present disclosure, the active compound is combined or formulated with appropriate pharmaceutically acceptable carriers, diluents, or excipients, and may be formulated into preparations in solid, semi-solid, liquid, or gaseous forms, such as tablets, capsules, powders, granules, ointments, solutions, suppositories, injections, inhalants, gels, microspheres, and aerosols. Routes of administration include oral, intraperitoneal, transdermal, subcutaneous, intravenous or intramuscular injection, inhalation, topical, intralesional, infusion; liposome-mediated delivery; local, intrathecal, periodontal pocket, rectal, endobronchial, nasal, transmucosal, intestinal, ocular or otic delivery, or any other method known in the art, all of which can achieve the prevention and/or treatment of fibrosis or related diseases or conditions.

### BRIEF DESCRIPTION OF THE DRAWINGS

Figure 1 shows H&E and Masson staining images of lung tissue;
Figure 2 shows the scoring of the degree of fibrosis in mouse lung tissue (* indicates comparison with the normal control group; # indicates comparison with the vehicle-treated group);
Figure 3 shows the TG detection results of the normal control group, the NASH group, and the 60 nM group of Compound 12 p-toluenesulfonate;
Figure 4 shows the inflammatory factor detection results of the normal control group, the NASH group, and the 60 nM group of Compound 12 p-toluenesulfonate;
Figure 5 shows the results of H&E staining, Sirius Red staining, and COL1 and α-SMA immunofluorescence staining for each group;
Figure 6 shows the ratio of the steatotic area to the total stained area of the slides after H&E staining;
Figure 7 shows the ratio of the fibrotic area to the total stained area of the slides after Sirius Red staining.

### DETAILED DESCRIPTION

The technical solutions of the present disclosure will be further described in detail below in conjunction with specific embodiments. It should be understood that the following embodiments are only illustrative for explaining the present disclosure and should not be construed as limiting the scope of protection of the present disclosure. All technologies realized based on the above content of the present disclosure are included within the scope intended to be protected by the present disclosure.

Unless otherwise specified, the materials and reagents used in the following embodiments are commercially available products or can be prepared by known methods.

### Compound Preparation Examples

In some embodiments of the present disclosure, the compound of formula (I) includes the following structure. These compounds can be prepared according to the method disclosed in WO2021098691A1 or the following examples.

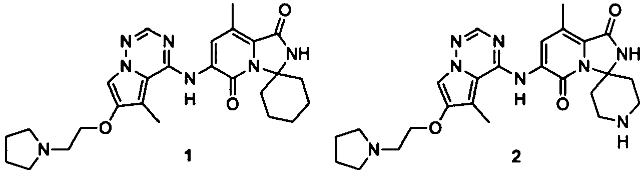

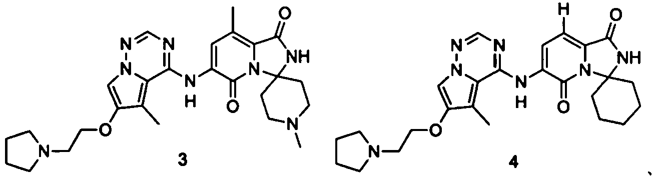

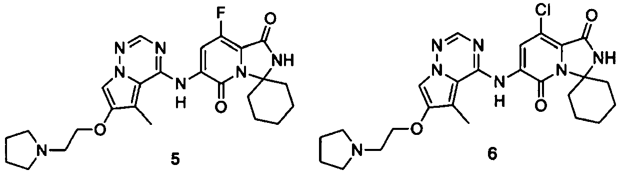

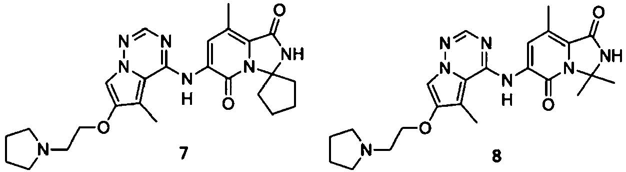

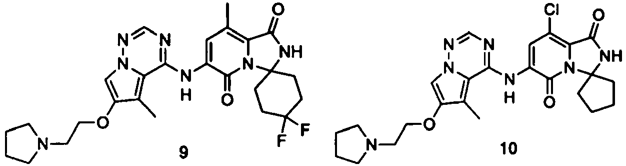

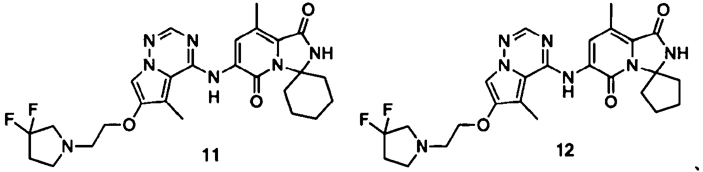

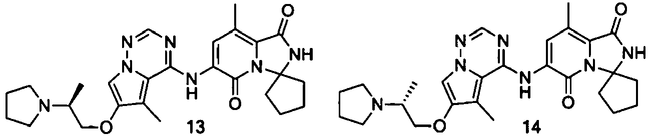

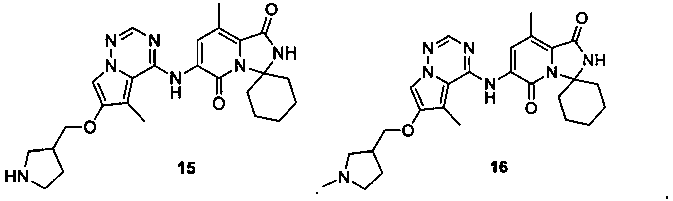

### Example 1

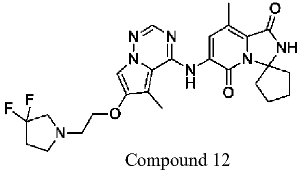

### Synthetic route:

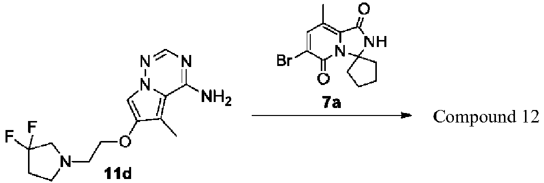

### Step 1

The trifluoroacetate of compound 11d (90 mg, 219 µmol) and compound 7a (72 mg, 241 µmol) were dissolved in anhydrous dioxane (2 mL), then cesium carbonate (250 mg, 766 µmol) and methanesulfonato(2-dicyclohexylphosphino-3,6-dimethoxy-2',4',6'-triisopropyl-1,1'-biphenyl)(2'-amino-1,1'-biphenyl-2-yl)palladium(II) (20 mg, 21.9 µmol) were added, and the reaction mixture was stirred at 105°C for 12 hours under nitrogen protection. The reaction solution was concentrated under reduced pressure and purified by column chromatography (10:1, dichloromethane/methanol, Rf = 0.3) to obtain the crude compound. A mixed solution of methanol and ethanol (4/1, 10 mL) was added to the crude product and stirred at 20°C for 16 hours. The mixture was filtered, and the filter cake was washed with methanol (2 mL × 2) and water (2 mL × 2), then dried to obtain Compound 12.

MS-ESI calculated value [M+H] ⁺ 514, found 514. ¹H NMR (400 MHz, DMSO-d₆) δ = 10.00 (s, 1H), 8.84 (s, 1H), 8.64 (s, 1H), 8.08 (s, 1H), 7.70 (s, 1H), 4.08 (t, J = 5.6 Hz, 2H), 3.00 (t, J = 13.5 Hz, 2H), 2.91-2.78 (m, 6H), 2.47 (s, 3H), 2.46 (s, 3H), 2.31-2.18 (m, 2H), 2.04-1.92 (m, 2H), 1.91-1.78 (m, 2H), 1.76-1.62 (m, 2H).

Compound 12 (2 g, 3.89 µmol) was mixed with hexafluoroisopropanol (40 mL) and stirred uniformly. p-Toluenesulfonic acid monohydrate (814.89 mg, 4.28 mmol) was added to the solution, and the reaction mixture was stirred at 40°C for 3 hours. The reaction solution was then added dropwise into isopropanol (160 mL), filtered, and the filter cake was vacuum-dried to obtain the corresponding p-toluenesulfonate salt of Compound 12. ¹H NMR (400 MHz, DMSO-d₆) δ = 10.02 (s, 1H), 8.86 (br s, 1H), 8.64 (s, 1H), 8.11 (s, 1H), 7.78 (s, 1H), 7.48 (d, J = 8.0 Hz, 2H), 7.10 (d, J = 8.0 Hz, 2H), 4.31 (br d, J = 4.4 Hz, 2H), 4.08-3.62 (m, 6H), 2.89-2.78 (m, 2H), 2.72-2.57 (m, 2H), 2.51 (br s, 3H), 2.46 (s, 3H), 2.28 (s, 3H), 1.95-1.97 (m, 2H), 1.89-1.79 (m, 2H), 1.73-1.64 (m, 2H). MS-ESI calculated value [M+H]⁺ 514, found 514.

### Biological Activity Test

### Biological Example 1: Evaluation of the therapeutic effect of the compounds of the present disclosure in bleomycin-induced pulmonary fibrosis

### 1.1 Establishment of the bleomycin-induced pulmonary fibrosis model

Experimental preparation: C57BL/6 mice, 8-10 weeks old, female, body weight approximately 18-22 g. Bleomycin. 1% sodium pentobarbital, sterile normal saline, insulin syringe.

Drug: p-toluenesulfonate salt of Compound 12 (10 mg/mL). The specific drug preparation method was as follows: 282.87 mg of the p-toluenesulfonate salt powder of Compound 12 was weighed and mixed with 1.050 mL of DMSO solution (preheated to 45°C). The mixture was placed in an ultrasonic water bath until completely dissolved. Subsequently, 19.95 mL of mixed solvent (comprising 1.995 mL of Solutol, 1.995 g of hydroxypropyl-β-cyclodextrin, and 15.96 mL water ) was added. The resulting mixture was sonicated again until completely dissolved to obtain a working solution with a concentration of 10 mg/mL. 1. After the mice begin to adapt to the environment, the experiment is started. The experimental animals are divided into five groups: normal control group, model group (Bleomycin group), low-dose treatment group (25 mg/kg), high-dose treatment group (50 mg/kg), and vehicle treatment group. The model group is used to compare with the normal group to determine whether the model establishment is successful, and the vehicle treatment group is used to reflect the effect of the vehicle on pulmonary fibrosis in mice and to exclude interference. 10 animals per group; their condition was observed during the period and samples were collected for subsequent experiments.

2. Half an hour before surgery, mice were given an intraperitoneal injection of 1% sodium pentobarbital (solvent: normal saline), 50 mg/kg; for mice weighing 18 g-22 g, 90-110 µl per mouse. After the mice were anesthetized, they were fixed in position and disinfected.

3. Bleomycin was used to establish the model at a dose of 3 mg/kg, 50 µl per mouse, administered via intratracheal instillation through the oral cavity. During the process, mouth breathing was maintained in the mice to ensure inhalation of the liquid, thereby establishing a bleomycin-induced mouse pulmonary fibrosis model.

4. After the liquid was instilled, the mouse was held upright to allow the drug to distribute evenly from the trachea and bronchi to the entire lung. Unobstructed breathing was maintained and the mouse was allowed to recover naturally.

5. This model has one endpoint: 28 days. According to the time point, the mice were euthanized and samples were collected for subsequent experiments; the day of modeling is recorded as day 1.

### 1.2 Lung inflammation scoring

1. Fresh mouse tissues were immersed in 4% paraformaldehyde solution for fixation. After 48 hours, they were placed in embedding cassettes and rinsed overnight under running tap water.
2. The tissue was placed in 75% ethanol for 30 minutes.
3. The tissue was placed in 85% ethanol for 30 minutes.
4. The tissue was placed in 95% ethanol twice, for a total of one hour.
5. The tissue was placed in 100% ethanol three times, for a total of one hour.
6. After removal, the tissue was placed in xylene twice, for a total of one hour.
7. Paraffin infiltration twice for a total of one hour; after infiltration, the embedding cassette was placed in fresh paraffin for embedding.
8. After embedding, the paraffin block was cooled on ice. The blocks were first trimmed on a microtome to expose the tissue, and 3-5 µ m thick sections were subsequently cut using a fresh blade. The sections were then floated on a water bath maintained at 42°C to flatten. Once fully expanded, the sections were mounted onto glass slides and baked at 65°C. After the sections were completely dried, the slides were removed and stored for subsequent procedures.

### Section hydration

1. The slides were placed on a slide rack and baked at about 65°C for 2 hours; they were removed after the paraffin on the sections had melted and dried.
2. The sections were placed in xylene twice, for a total of one hour.
3. The sections were transferred to 100% ethanol twice, two minutes each time.
4. The sections were transferred to 95% ethanol once, two minutes.
5. The sections were transferred to 85% ethanol once, two minutes.
6. The sections were transferred to 75% ethanol once, two minutes.
7. After the above steps were completed, the sections were transferred to distilled water and rinsed for two minutes.

### HE Staining

1. After hydration was completed, the sections were stained with Mayer's hematoxylin for one minute.
2. The sections were rinsed in tap water twice, three minutes each time.
3. The sections were placed in 75% acid alcohol for about two minutes.
4. The sections were rinsed with tap water for one minute.
5. The sections were counterstained with eosin for about one minute.
6. The sections were rinsed with tap water for one minute.
7. The sections were placed in 85% ethanol for about two minutes.
8. The sections were placed in 95% ethanol for about two minutes.
9. The sections were placed in 100% ethanol for about two minutes.
10. After the above steps were completed, the sections were placed in xylene for 5-10 minutes.
11. The sections were mounted with neutral resin.

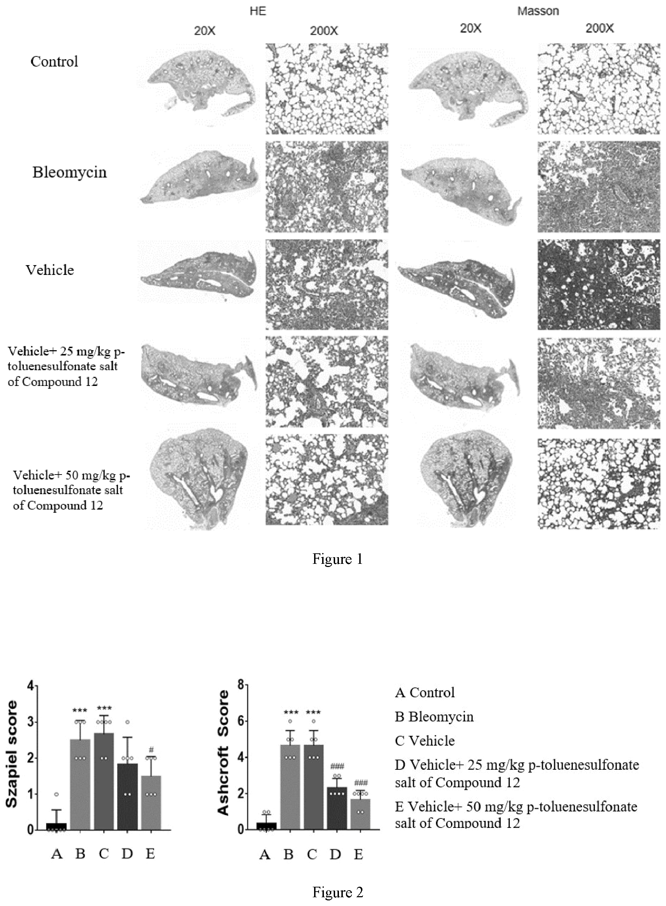

After scanning 5 µm-thick paraffin-embedded lung tissue sections stained with HE, at least two independent scorers select five representative fields and score the degree of fibrosis according to the scoring criteria shown in Table 1. The average value is taken as the Szapiel Score of the sample. The results are shown in Figure 1.

As shown in Figure 2, the Szapiel Score of lung tissue fibrosis among the groups of mice was compared and analyzed. The results showed that, compared with the normal control group, the model group (Bleomycin group) had a significantly higher score, with a significant difference (p value<0.05), indicating successful model establishment. Compared with the vehicle treatment group, the high-dose group (50 mg/kg) of Compound 12 p-toluenesulfonate showed a significantly lower score, with statistical significance (p value<0.05).

| Szapiel scoring criteria | |
|---|---|
| 0 points | No injury or areas of fibrotic alveolitis |
| 1 point | Injury or fibrotic alveolitis area <25% |
| 2 points | Injury or fibrotic alveolitis area occupying 25%-50% |
| 3 points | Injury or fibrotic alveolitis area >50% |

### 1.3 Lung Fibrosis Grading Score

The Ashcroft Scoring System was invented by T. Ashcroft in 1988 and has been widely used to grade the degree of fibrosis in animal and human tissues (e.g., Lancet Respir Med, 2020, PMID: 32061334 & Eur Respir J, 2009, PMID: 19460787).

### Masson staining

1. The hydrated sections were placed in Masson compound neutral staining solution for 5 minutes;
2. The sections were rinsed in 0.2% acetic acid aqueous solution for about 2 minutes;
3. The sections were immersed in 8% phosphotungstic acid for about 10 minutes;
4. The sections were rinsed in 0.2% acetic acid aqueous solution for about 2 minutes;
5. The sections were stained with 0.2% aniline blue solution for 5 minutes;
6. The sections were rinsed in 0.2% acetic acid aqueous solution for about 2 minutes, twice;
7. The sections were placed in 85% ethanol for about two minutes.
8. The sections were placed in 95% ethanol for about two minutes.
9. The sections were placed in 100% ethanol for about two minutes.
10. After the above steps were completed, the sections were placed in xylene for 5-10 minutes.
11. The sections were mounted with neutral resin.

After scanning the 5 µm thick paraffin-embedded lung tissue sections stained with Masson, at least two independent scorers select five representative fields (10× or 20× magnification) and score the degree of fibrosis according to the scoring criteria shown in Table 1. The final average value is taken as the Ashcroft Score for the sample. The scoring results are shown in Figure 2.

As shown in Figure 2, the Ashcroft Scores of lung tissue fibrosis in mice from each group were compared and analyzed. The results showed that, compared with the normal control group, the model group (Bleomycin group) had significantly increased scores with a significant difference (p value <0.05), indicating successful model establishment. Compared with the vehicle-treated group, the low-dose group (25 mg/kg) and the high-dose group (50 mg/kg) receiving Compound 12 p-toluenesulfonate salt showed significantly lower scores, and the differences were statistically significant (p value <0.05).

| Score | Histological features |
|---|---|
| 0 | Normal lung |
| 1 | Mild fibrotic thickening visible in alveolar walls or bronchial walls |
| 2 | When it is impossible to distinguish between 1 point and 3 points, record 2 points |
| 3 | Moderate fibrotic thickening can be seen in the alveolar wall or bronchial wall, with no obvious destruction of lung tissue structure |
| 4 | When it is impossible to distinguish between 3 points and 5 points, record 4 points |
| 5 | The lung tissue structure shows obvious destruction, with fibrotic strands or small fibrotic clusters and honeycomb lung |
| 6 | When it is impossible to distinguish between 5 points and 7 points, record 6 points |
| 7 | The lung tissue structure is severely destroyed, with large areas of fibrosis |
| 8 | Fibrotic areas fill the entire field of view |

### Biological Example 2: Pharmacodynamic evaluation of the compound of the present disclosure in a NASH model associated fibrosis

### 2.1 In vitro 3D liver model construction (reference: CN115386533A)

### 2.1.1 D-2~D0 modeling:

Four types of human primary cells were used for modeling, including human primary hepatocytes (PHH), human primary liver sinusoidal endothelial cells (LSEC), human primary hepatic stellate cells (HSC), and human primary Kupffer cells (KC). The total number of cells in each model was 3000.

The cell connector NAC-Linker A was mixed with hepatocytes and incubated at room temperature for 30 minutes so that NAC-Linker A was fixed on the cell membrane surface. At the same time, the cell connector NAC-Linker B was mixed with the mixture of intrahepatic non-parenchymal cells and incubated at room temperature for 30 minutes so that NAC-Linker B was fixed on the cell membrane surface; (2) after incubation, the hepatocytes carrying NAC-Linker A and the intrahepatic non-parenchymal cells carrying NAC-Linker B were mixed evenly, and 20-30 µl droplets were prepared on the lid of a culture plate using a pipette. PBS solution and liver physiological culture medium (Puheng Biomedicine (Shanghai) Co., Ltd., liver physiological culture medium (human), catalog No. M0001) were added to the wells of the culture plate. The prepared culture plate lid was inverted and placed onto the culture plate to form hanging drops. The culture plate with hanging drops was placed upside down in a 37°C incubator for 12-24 hours. Under the curvature of the lower surface of the hanging drops and gravity, complementary DNA pairing in the NAC-Linker formed connections to generate a 3D liver organoid structure.

### 2.1.2 Experimental grouping and drug treatment

After successful modeling on Day 0, the samples were divided into 5 groups according to different treatments, with 9 samples in each group. Among them, the normal control group continued to be treated with liver physiological culture medium; the NASH group was treated with NASH induction culture medium (Puheng Biomedicine (Shanghai) Co., Ltd., NASH induction culture medium (human), catalog No. MI001); the low-dose group of the p-toluenesulfonate salt of Compound 12 was treated with NASH induction culture medium and 10 nM of the p-toluenesulfonate salt of Compound 12; the medium-dose group of the p-toluenesulfonate salt of Compound 12 was treated with NASH induction culture medium and 30 nM of the p-toluenesulfonate salt of Compound 12; the high-dose group of the p-toluenesulfonate salt of Compound 12 was treated with NASH induction culture medium and 60 nM of the p-toluenesulfonate salt of Compound 12.

Dosing: Starting on Day 0, Compound 12 p-toluenesulfonate was added to the low-dose, medium-dose, and high-dose groups respectively. Culture conditions remained unchanged. A complete medium change was performed every 2 days, replacing with fresh medium corresponding to each group and the same concentration of the drug.

### 2.1.3 Quality Control

Quality control testing of the model was performed on Day 0, Day 5, and Day 9. Day 0: H&E staining confirmed that when model construction was completed, the cells showed no necrosis and the model exhibited no pathological changes. Day 5: H&E staining and Sirius Red (SR) staining were performed to determine the degree of fibrosis in the model on Day 5. Day 9: H&E staining and SR staining were performed to determine the degree of steatosis in the NASH model on Day 9.

### 2.2 Experimental Tests and Results

### 2.2.1 Quality Control Test Results

Day 0: H&E staining results showed that the cells were in a normal state with no obvious necrotic areas, and the model showed no pathological changes, indicating it was suitable for subsequent experiments.

Day 5: H&E staining results showed that cells in the normal control group were in a normal state, while ballooning degeneration was observed in the NASH group, indicating a trend toward steatosis; SR staining results showed fibrotic accumulation in the NASH group.

Day 9: H&E staining results showed that the cells were in a normal state, while obvious ballooning degeneration was observed in the NASH group with significant steatosis, indicating suitability for subsequent experiments; SR staining results showed normal expression of fibrotic lesions in the model.

### 2.2.2 TG Detection

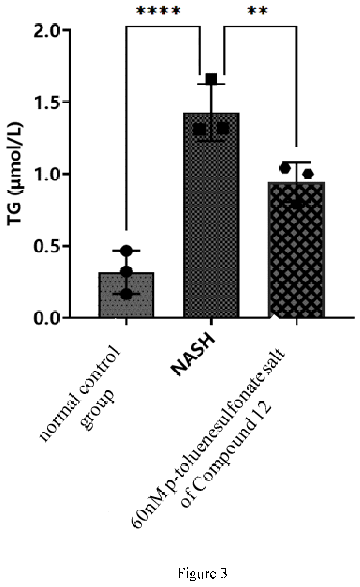

Triglyceride (TG) detection was performed on Day 10. Liver spheroids were ground in cell lysis buffer using a grinding pestle, centrifuged, and the supernatant was collected for enzymatic assay. As shown in Figure 3, the TG level in the 60 nM group of Compound 12 p-toluenesulfonate showed a significant difference (p<0.05) compared with the NASH group.

### 2.2.3 Cytokine Detection (IL-6)

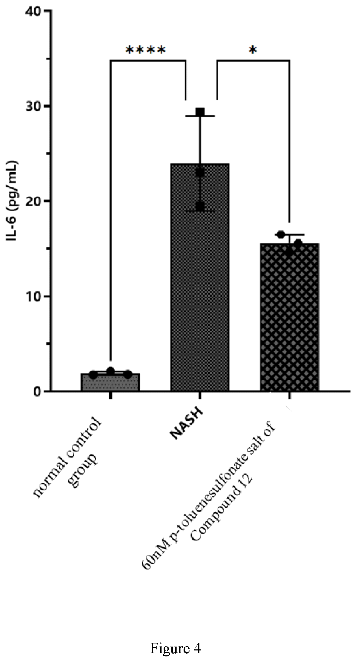

The culture medium supernatant was collected and tested using an IL-6 detection kit according to the manufacturer's instructions, with measurement performed using a microplate reader. The results are shown in Figure 4. The inflammatory cytokine level in the 60 nM group of Compound 12 p-toluenesulfonate was improved compared with the NASH group, and the data for the 60 nM group showed a significant difference compared with the NASH group (p<0.05).

### 2.2.4 H&E Staining

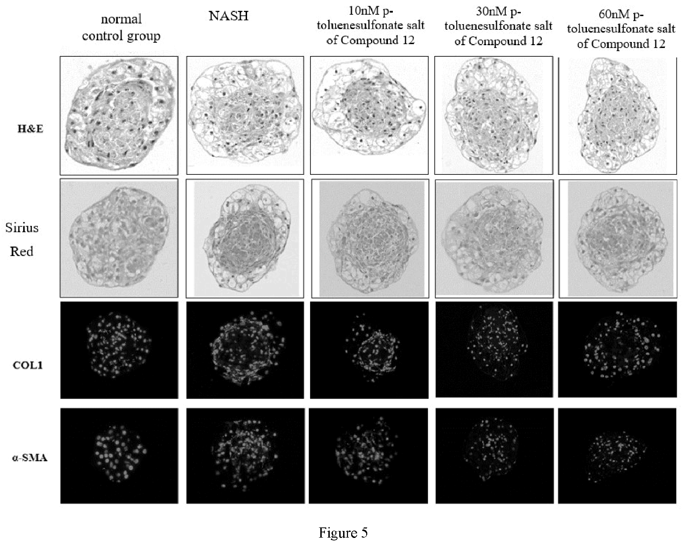

Liver spheroids were prepared as paraffin sections and subjected to H&E staining. The staining results are shown in Figure 5.

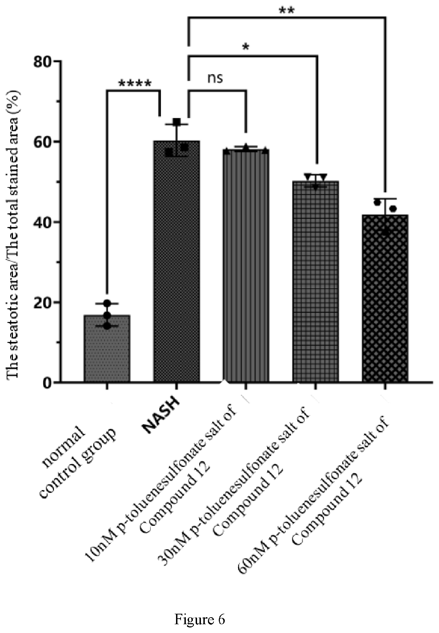

Using ImageJ software, the steatotic area on the slides after H&E staining was quantified, and the ratio of the steatotic area to the total area of each slide was calculated (results are shown in Figure 6).

H&E staining results showed that, compared with the NASH group, the drug group of Compound 12 p-toluenesulfonate at a dose of 10 nM showed improvement in hepatocyte steatosis and ballooning degeneration; in the drug groups at doses of 30 nM and 60 nM, the H&E staining results showed significant histological improvement, specifically manifested as a marked reduction in hepatocyte steatosis and a significant decrease in the number of ballooned hepatocytes. These results indicate that Compound 12 p-toluenesulfonate has a significant therapeutic effect in inhibiting the pathological progression of NASH.

### 2.2.5 Sirius Red Staining

Liver spheroids were paraffin-embedded and sectioned, stained with Sirius Red, mounted, and photographed, and the results are shown in Figure 5.

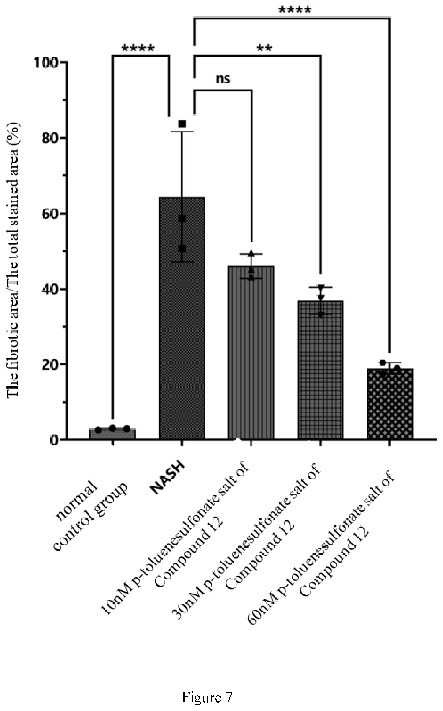

Using ImageJ software, the fibrosis area on the slides after Sirius Red staining was quantified, and the ratio of the fibrosis area to the total area of each slide was calculated (Figure 7).

Sirius Red staining was used to evaluate the effect of the drug on liver fibrosis, and the results showed that the degree of liver fibrosis in the Compound 12 p-toluenesulfonate 10 nM group showed a trend of improvement compared with the normal control group. In the staining results of the Compound 12 p-toluenesulfonate 30 nM group and the Compound 12 p-toluenesulfonate 60 nM group, the fibrosis area in the liver spheroids of each group showed significant differences compared with the NASH group, indicating that the above two groups exhibited significant therapeutic effects on liver fibrosis induced by NASH, and the higher the dose of Compound 12 p-toluenesulfonate, the better the therapeutic effect.

### 2.2.6 Immunofluorescence Staining

Liver spheroids were paraffin-embedded and sectioned, subjected to immunofluorescence staining, mounted, and photographed under a fluorescence microscope, and the results are shown in Figure 5

The results of COL1 and α-SMA immunofluorescence staining showed that Compound 12 p-toluenesulfonate has a therapeutic effect on liver fibrosis and exhibits dose dependence.

The implementation modes of the technical solution of the present disclosure have been illustratively described above. It should be understood that the scope of protection of the present disclosure is not limited to the above embodiments. Any modifications, equivalent substitutions, improvements, etc. made by those skilled in the art within the spirit and principles of the present disclosure shall fall within the scope of protection of the claims of this application.

## Claims

1. Use of a compound of formula (I) or a pharmaceutically acceptable salt thereof in the preparation of a medicament for preventing and/or treating fibrosis or related diseases, 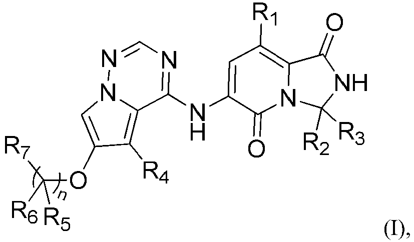
wherein R₁ is selected from H, F, Cl, Br, or C₁₋₃ alkyl;
R₂ and R₃ are each independently selected from H or C₁₋₃ alkyl, wherein the C₁₋₃ alkyl is optionally substituted with 1, 2, or 3 substituents independently selected from F, Cl, Br, or I;
or R₂ and R₃ together with the carbon atom to which they are attached form cyclopentyl, cyclohexyl, or piperidinyl, wherein the cyclopentyl, cyclohexyl, and piperidinyl are optionally substituted with 1, 2, or 3 Rₐ;
each Rₐ is independently selected from H, F, Cl, Br, or C₁₋₃ alkyl;
R₄ is selected from H, F, Cl, Br, or C₁₋₃ alkyl;
R₅ and R₆ are each independently selected from H, F, Cl, Br, I, or C₁₋₃ alkyl;
R₇ is pyrrolidinyl, wherein the pyrrolidinyl is optionally substituted with 1, 2, or 3 R_{b};
each R_{b} is independently selected from H, F, Cl, Br, I, or C₁₋₃ alkyl, wherein the C₁₋₃ alkyl is optionally substituted with 1, 2, or 3 substituents independently selected from F, Cl, Br, or I;
n is 1 or 2.

2. The use according to claim 1, **characterized in that** Rₐ is independently selected from H, F, Cl, Br, - CH₃, or -CH₂CH₃;
Preferably, R₂ and R₃ are each independently selected from H, -CH₃, or -CH₂CH₃;
Preferably, R₂ and R₃ together with the carbon atom to which they are attached form 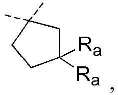 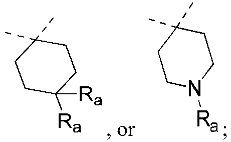
Preferably, R₂ and R₃ together with the carbon atom to which they are attached form 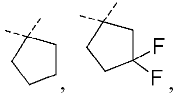 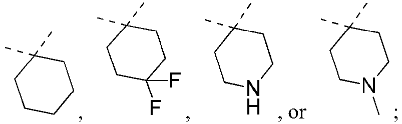
Preferably, 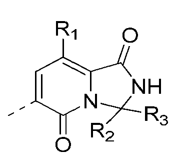 is selected from 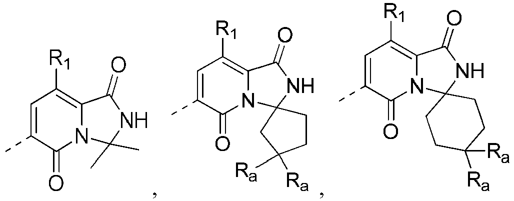 or 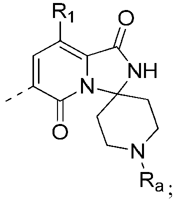
Preferably, 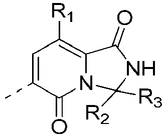 is selected from 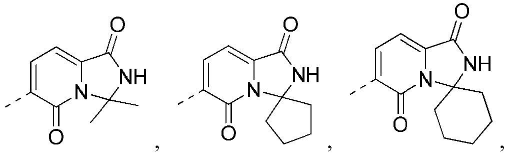 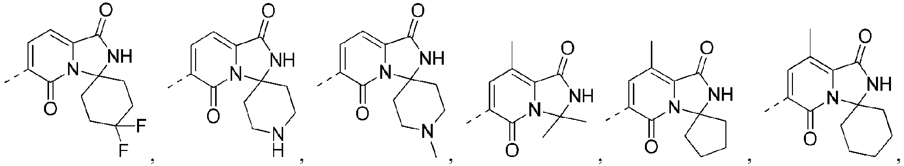 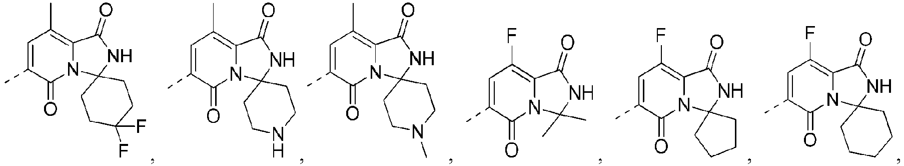 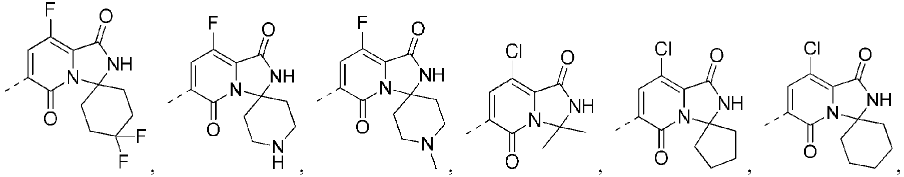 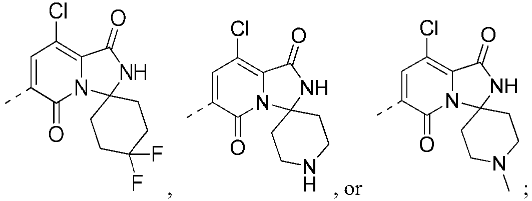
Preferably, the compound of formula (I) has the structure shown in any one of structural formulas (I-1) ~ (I-4): 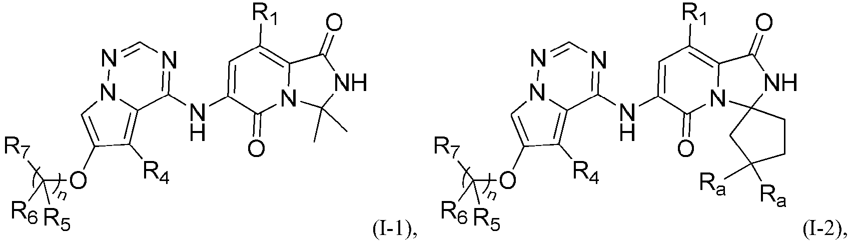 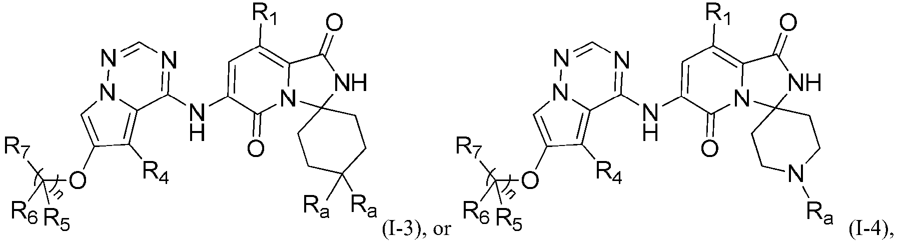
wherein R₁, R₄, R₅, R₆, R₇, Rₐ, and n are as defined above or in claim 1;
Preferably, each R_{b} is independently selected from H, F, Cl, Br, I, 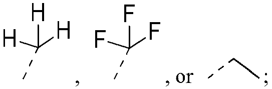
Preferably, R₇ is selected from 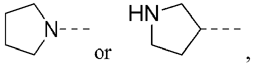 wherein 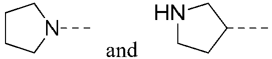 are optionally substituted with 1 or 2 R_{b};
Preferably, R₇ is selected from 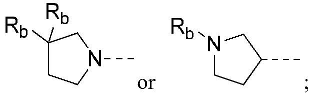
Preferably, R₇ is selected from 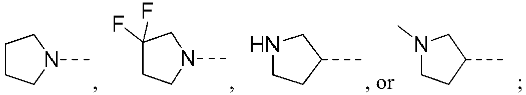
Preferably, R₄ is selected from H or -CH₃;
Preferably, the compound of formula (I) has the structure shown in any one of structural formulas (I-5) ~ (I-9): 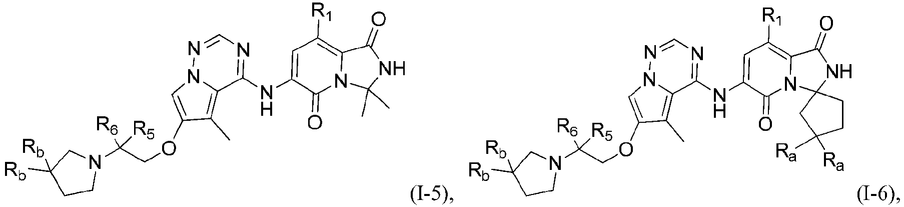 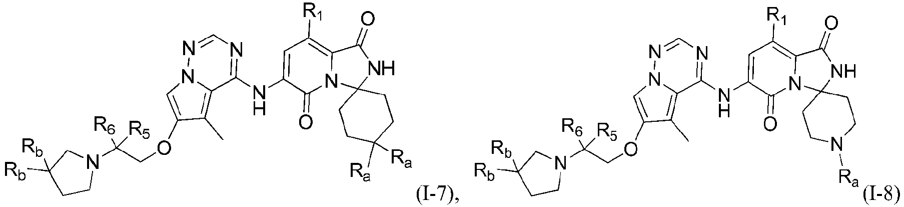 or 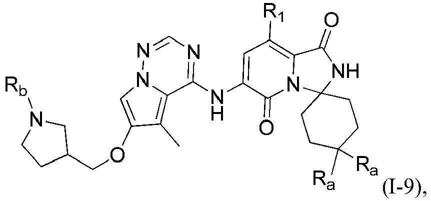
wherein R₁, R₅, R₆, Rₐ, and R_{b} are as defined above or in claim 1;
Preferably, R₁ is selected from H, F, Cl, or 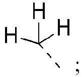
Preferably, R₅ and R₆ are each independently selected from H or 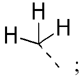
Preferably, R₁ is C₁₋₃ alkyl, such as methyl;
Preferably, R₂ and R₃ together with the carbon atom to which they are attached form 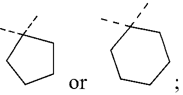
Preferably, R₄ is C₁₋₃ alkyl, such as methyl;
Preferably, R₅ and R₆ are each independently selected from H or methyl; n is 2;
Preferably, 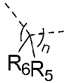
is 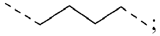
Preferably, R₇ is 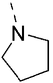
substituted with 1, 2, or 3 H, F, Cl, or methyl, for example 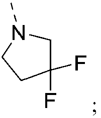
Preferably, the compound represented by formula (I) is selected from the following structures:

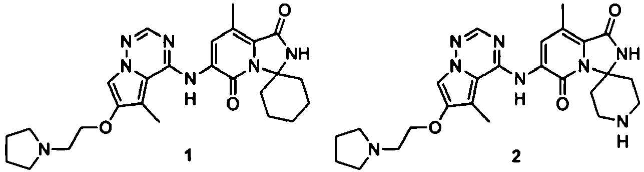

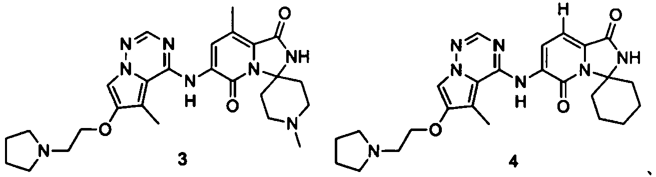

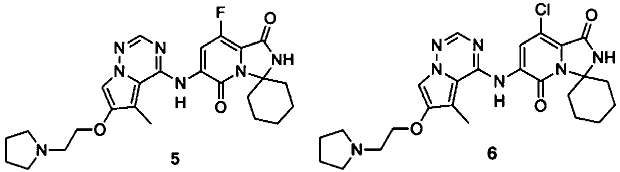

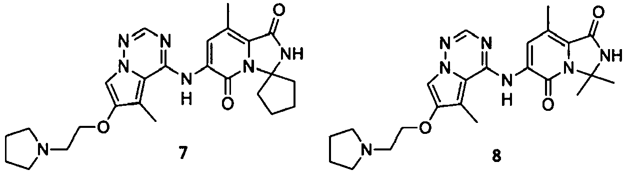

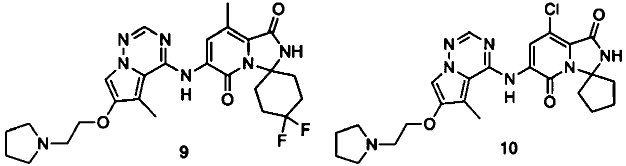

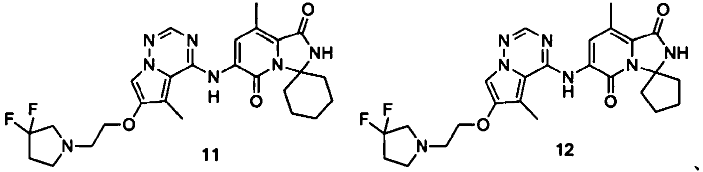

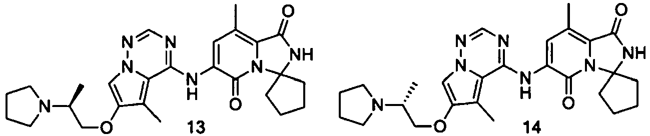

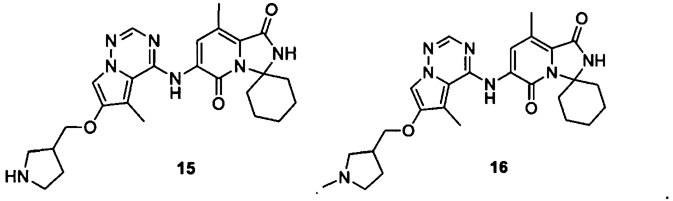

3. The use according to claim 1 or 2, **characterized in that** the pharmaceutically acceptable salt is a salt formed between the compound represented by formula (I) and an inorganic acid, wherein the inorganic acid includes, for example, hydrochloric acid, hydrobromic acid, nitric acid, carbonic acid, bicarbonate, phosphoric acid, hydrogen phosphate, dihydrogen phosphate, sulfuric acid, hydrogen sulfate, hydroiodic acid, phosphorous acid, etc.; and organic acid salts, wherein the organic acids include acids such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; also including salts of amino acids (such as arginine, etc.), as well as salts of organic acids such as glucuronic acid; preferably the hydrochloride or p-toluenesulfonate salt of the compound represented by formula (I).

4. The use according to any one of claims 1-3, **characterized in that** the fibrosis or related disease or condition is selected from pulmonary fibrosis, renal fibrosis, myelofibrosis, cystic fibrosis, oral mucosal fibrosis, hepatic fibrosis, biliary fibrosis, myocardial fibrosis, skin fibrosis, ocular fibrosis, and pancreatic fibrosis.

5. The use according to any one of claims 1-3, **characterized in that** the fibrosis or related disease or condition is selected from inflammatory diseases, for example selected from pneumonia, hepatitis, nephritis, myocarditis, and pancreatitis.

6. The use according to any one of claims 1-3, **characterized in that** the fibrosis or related disease or condition is selected from liver-related diseases, for example hepatitis, liver cirrhosis, liver injury, or liver failure.

7. The use according to any one of claims 1-3, **characterized in that** it is selected from nonalcoholic fatty liver disease or nonalcoholic steatohepatitis.

8. The use according to any one of claims 1-3, **characterized in that** the fibrosis or related disease or condition is selected from progressive fibrosing interstitial lung disease (PF-ILD), particularly diseases presenting pulmonary fibrosis manifestations, such as idiopathic pulmonary fibrosis (IPF), systemic sclerosis-associated ILD (SSc-ILD), connective tissue disease-associated ILD (CTD-ILD), rheumatoid arthritis-associated ILD (RA-ILD), chronic fibrosing hypersensitivity pneumonitis (HP), idiopathic nonspecific interstitial pneumonia (iNSIP), unclassifiable idiopathic interstitial pneumonia (IIP), environmental/occupational fibrotic lung disease, interstitial pneumonia with autoimmune features (IPAF), and sarcoidosis; or the fibrosis or related disease or condition is selected from muscular dystrophy, fibromatosis, and myelofibrosis, preferably selected from Duchenne muscular dystrophy, Dupuytren's contracture, and primary myelofibrosis (PMF).

9. A method for preventing and/or treating fibrosis or related diseases or conditions, the method comprising administering to a patient a therapeutically effective amount of the compound of formula (I) or a pharmaceutically acceptable salt thereof.
